(19) Europäisches Patentamt
European Patent Office
Office européen des brevets

(11) Publication number: 0 112 679
B1

(12) EUROPEAN PATENT SPECIFICATION

(45) Date of publication of patent specification: 30.04.86

(21) Application number: 83307557.5

(22) Date of filing: 13.12.83

(51) Int. Cl.[4]: C 07 C 69/76, C 07 C 67/36, C 07 C 69/78, C 07 C 69/92, C 07 C 65/11, C 07 C 69/94, C 07 C 63/06, C 07 D 333/40, C 07 C 63/36, C 07 D 333/38, C 07 C 63/26

# (54) Process for the preparation of esters or salts of aromatic or etheroaromatic acids.

(30) Priority: 14.12.82 IT 2472382

(43) Date of publication of application:
04.07.84 Bulletin 84/27

(45) Publication of the grant of the patent:
30.04.86 Bulletin 86/18

(84) Designated Contracting States:
BE CH DE FR GB LI NL

(56) References cited:
FR-A-2 286 126
US-A-2 565 462

(73) Proprietor: Montedison S.p.A.
31, Foro Buonaparte
I-20121 Milan (IT)

(72) Inventor: Foa, Marco
19, via del Sabbione
Novara (IT)
Inventor: Francalanci, Franco
7, via Ferraris
Novara (IT)
Inventor: Gardano, Andrea
11, corso Roma
Trino Vercellese (Vercelli) (IT)
Inventor: Bencini, Elena
170, c.so XXIII marzo
Novara (IT)

(74) Representative: Whalley, Kevin et al
Marks & Clerk 57/60 Lincoln's Inn Fields
London WC2A 3LS (GB)

EP 0 112 679 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the preparation of esters or salts of aromatic or etheroaromatic acids.

More particularly, the invention relates to a process for the preparation of esters or salts of aromatic or etheroaromatic acids of formula (I):

$$Y—Ar—CO—OR \quad (I)$$

wherein:

Ar represents an aromatic group constituted by one or more benzene rings, optionally condensed, or by an etheroaromatic nucleus optionally condensed with one or more benzene rings;

Y represents zero, one or more substituents, which may be the same or different, selected from a halogen; an alkyl group having up to 6 carbon atoms; an alkoxy group having up to 5 carbon atoms; an ester group —COOR′ where R′ contains up to 5 carbon atoms; an hydroxyl group; a phenyloxy group optionally substituted with groups inert under reaction conditions; a trifluoromethyl group; a nitrile group; an amidic group ($—CONH_2$); an acetamidic group ($—NH—CO—CH_3$) or an acyl group —CO—R″ where R″ represents a hydrocarbon group having up to 8 carbon atoms; and

R represents an alkyl group $R_1$ having up to 5 carbon atoms or an alkali or alkaline-earth metal M.

The corresponding aromatic or etheroaromatic acids can be obtained by further treatment of the esters and salts obtained by the process of the invention, by following known procedures.

Such esters and their corresponding acids find various applications. They can be used, for example, in the preparation of alkydic resins (benzoic acid), of dyes and pigments (naphthoic acids), of azo-dyes (anthranilic acid) and in the field of plasticizing agents (esters of phthalic acid with higher alcohols).

Processes for the preparation of carboxylic arylic acids and their esters by carbonylation of aryl halides with carbon monoxide in the presence of a transition metal, for example Ni, Pd or Pt, are known. The reaction generally requires the use of high pressures and temperatures; however, these difficult working conditions adverseley affect the economy of such processes, making them unsuitable for industrial application.

A variant of the above mentioned processes comprises the use of $Ni(CO)_4$ catalysts and of special dipolar protonless solvents associated with basic compounds which allow milder working conditions to be utilized. The use of $Ni(CO)_4$ as catalyst, considering its high volatility and toxicity, is however disadvantageous; another disadvantage of the process lies in the use of expensive solvents.

It has also been proposed to perform carbonylation of aromatic halides in the presence of palladium catalysts with phosphinic ligands, both in homogeneous phase and in phase-transfer conditions. The high cost of the catalyst and the use of phosphinic ligands make this process economically disadvantageous.

Recently, for such carbonylation reaction of aromatic halides, the use of catalytic systems constituted by cobalt salts, alcoholates and sodium hydride has been reported; the use of cobalt carbonyl with simultaneous irradiation with UV light has also been proposed: these are mainly scientific methods for which, at the present time, it is difficult to envisage industrial application.

The present invention aims to provide a simple and economic process for the preparation of esters or salts of aromatic or etheroaromatic acids of formula (I), avoiding the drawbacks of the above mentioned processes. Another aim is to provide a process which may be performed at or near ambient temperature and at atmospheric pressure. It is also desirable that the process may utilize a readily preparable non-toxic catalyst, and that the process may give high selectivity with respect to the desired product.

The present invention provides a process for the preparation of esters or salts of aromatic or etheroaromatic acids of formula Y—Ar—CO—OR (I), where Ar, Y and R are as previously defined, wherein a halide of formula (II):

$$Y—Ar—X \quad (II)$$

where Ar and Y have the meanings as previously defined and where X is Cl, Br or I, is reacted with carbon monoxide in an alcoholic solvent $R_1OH$ (where $R_1$ is an alkyl group having up to 5 carbon atoms) at atmospheric pressure and at a temperature from −10° to 60°C in the presence of an acidity-acceptor compound and of a catalyst constituted by a cobalt complex of formula (III):

$$Z—Co(CO)_4 \quad (III)$$

where Z is a group selected from $CH_3$; $CH_2F$; $CHF_2$; $CF_3$; $CH_2CN$; $CH_2—COOR'''$ where R‴ is an alkyl group having up to 8 carbon atoms or a benzene group, the latter being optionally substituted with groups inert under reaction conditions; and $CH_2Ar'$ where Ar′ is an aromatic group constituted by from one to three benzene rings optionally condensed and optionally substituted with groups inert under reaction conditions, in particular with electron-attractor groups.

The reaction gives rise to the formation of esters or salts, as a function of the nature of the acidity-acceptor compound, according to the reactions hereunder indicated:

$$Y—Ar—X + CO + 2MOH \rightarrow Y—Ar—COOM + MX + H_2O \quad (1)$$

$$Y—ArX + CO + R_1OH + B \rightarrow Y—Ar—COOR_1 + BHX \quad (2)$$

$$Y—Ar—X + CO + R_1O^- \rightarrow Y—Ar—COOR_1 + X^- \quad (3)$$

In the reaction (I) the acidity-acceptor compound is an alkali or alkaline-earth oxide or hydroxide schematically represented by formula MOH, and a salt is obtained. NaOH, KOH and CaO are among the preferred MOH compounds.

In the reaction (2) a weaker acidity-acceptor compound B is used, preferably $Na_2CO_3$ or $K_2CO_3$, and an ester is obtained. In this case, the solvent $R_1OH$, where $R_1$ is an alkyl group having up to 5 carbon atoms, is one of the reagents.

In the reaction (3), the acidity-acceptor compound is an alcoholate $R_1O^-$ derived from alcohol $R_1OH$ used as solvent, and an ester is obtained.

The catalysts Z—$Co(CO)_4$ (III) are mainly known substances in themselves, described in the following publications:

W. Hieber and others, Z. Naturforsch. 13b, 192—4 (1958)
W. Hieber and others, Z. Naturforsch. 16b 229—31 (1961)
E. Lindner and others, Chem. Ber. 107, 1444—1455 (1974)
V. Galamb and others, Journal of Organometallic Chemistry 209 (1981) 183—195
V. Galamb and others, J. Chem. Soc., Chem. Commun., 1982, 487—488.

The process for the preparation of such catalysts is also described in these publications. Usually, they are obtained by reaction, generally in ether solvent, of the alkali metal salts of cobalt hydrocarbonyl with the corresponding organic halides of formula (IV):

$$Z{-}X \qquad (IV)$$

where Z and X are as previously defined, or with the anhydrides of formula (V):

$$Z{-}CO{-}O{-}CO{-}Z \qquad (V)$$

where Z is as previously defined.

When Z is a $CH_2$—COOR''' group, R''' is an alkyl group having up to 8 carbon atoms or a benzene group, the latter being optionally substituted with groups inert under reaction conditions; in the latter case, there are preferably from 1 to 3 inert groups, which may be the same or different, chosen in particular from among hydrocarbon groups having up to 8 carbon atoms, methoxy groups, phenoxy groups and ester groups containing up to 4 carbon atoms.

When Z is a $CH_2$—Ar' group, Ar' is an aromatic group constituted by from one to three benzene rings optionally condensed and optionally substituted with groups inert under reaction conditions, in particular with electron-attractor groups. The inert groups include those already defined hereinabove as well as the electron-attractor groups; the latter are preferred: for example, fluorine, $CF_3$, chromotricarbonyl $Cr(CO)_3$ group and, restricted to the case of benzene nuclei, chlorine are suitable (chlorine, in fact, is not reactive under reaction conditions when it is present on a benzene nucleus). The substituent groups may be the same or different, and preferably there are 1 to 3 of them for each benzene ring. Preferably, there are 1 or 2 benzene rings.

The starting halide Y—Ar—X (II) can contain more than one reactive halogen; in this case more than one —COOR group can replace a halogen. Hence, starting for example from p-dibromobenzene, a salt or an ester or terephthalic acid is obtained.

In the halide (II), Ar represents an aromatic group constituted by one or more benzene rings, optionally condensed, or an etheroaromatic nucleus optionally condensed with one or more benzene rings.

When Ar is an aromatic group with more than one benzene ring, the rings may be linked to each other for example through a direct carbon-carbon link; the rings can be condensed as well. Preferably the Ar group contains from one to three benzene rings.

When Ar is an etheroaromatic nucleus, one or more atoms chosen from N, O and S can be contained in it; for example, pyridine, furane and thiophene nuclei are suitable. The etheroaromatic nucleus can be condensed with one or more benzene nuclei; in this case there are preferably from 1 to 3 benzene nuclei.

In the halide (II), Y represents zero, one or more substituents, preferably from zero to 4 substituents.

When, in the halide (II), Y is a phenyloxy group optionally substituted with groups inert under reaction conditions, these inert groups, which may be the same or different, are chosen in particular from among hydrocarbon groups having up to 8 carbon atoms, methoxy groups, phenoxy groups and ester groups containing up to 4 carbon atoms; preferably there are from 1 to 4 inert groups.

Good results have been obtained for example with the following halides: 2-chloronaphthalene, 2-chlorothiophene, bromobenzene, 1-chloronaphthalene, 4-bromotoluene, 3-bromoanisol, p-dibromobenzene, 4-chlorobromobenzene, 2-chlorobromobenzene, 3-chlorobromobenzene, methyl 2-chlorobenzoate, 2-bromothiophene, 2-bromoacetoanilide, 3-bromopyridine, 3-bromofurane, and 2-hydroxy-6-bromonaphthalene.

The alcoholic solvent $R_1OH$ is an alkyl alcohol containing up to 5 carbon atoms. It is preferably chosen from methyl, ethyl and isopropyl alcohols.

The reaction is carried out at atmospheric pressure and at a temperature ranging from −10° to 60°C, preferably at a temperature ranging from 0° to 40°C. The catalyst, dissolved in a suitable solvent, is preferably added gradually into the reactor containing the other reagents and kept under CO atmosphere; ethers, in particular ethyl ether, hydrocarbons and $R_1OH$ alcohols are for example suitable solvents for the catalyst.

When $R_1O^-$ alcoholate is used, the latter can be prepared separately or produced *in situ* by reaction of $R_1OH$ alcohol with an alkali metal, in particular sodium.

The molar ratio between halide (II) and catalyst

(III) can vary within wide limits, for example 20:1 to 300:1, more preferably from 20:1 to 200:1.

The acidity-acceptor compound is generally introduced according to a substantially stoichiometric ratio with respect to halide (II), according to reaction (I), (2) or (3); however, an excess amount can be used as well, up to approximately 50% with respect to stoichiometry.

Complete reaction requires generally from 1 to 12 hours according to the temperature, the concentration of reagents, the nature of the halide (II) and the rate at which the solution containing the catalyst is added.

The process can be effectively performed as follows: the solvent, the acidity-acceptor compound and halide (II) are introduced, in a CO atmosphere into a reactor equipped with a stirrer, a thermometer and a condenser. Once the mixture has reached the desired temperature, the predetermined amount of catalyst, dissolved in a suitable solvent, is added gradually under stirring. During catalyst addition, the reaction progress is indicated by the absorption of carbon monoxide; the reaction ends when the absorption ceases.

The aromatic or etheroaromatic acid can be obtained from its salt or from its ester by known methods. For example, when the salt is obtained, water is added and the mixture is acidified with a mineral acid, for example HCl. The acid is extracted with a solvent, for example ether. The organic phase thus obtained is in its turn extracted with an aqueous solution of sodium bicarbonate, which is acidified and extracted with ether, which, after evaporation, supplies the acid that can be purified using known methods.

When an ester is obtained, it is for example possible to act as follows: the mixture is diluted with water, acidified and then extracted with ether. After ether evaporation, the ester is isolated using known techniques (for example by distillation or crystallization) or can be saponified in acid or basic ambient to give the acid.

The invention will be further described with reference to the following illustrative Examples.

Example 1

30 ml methyl alcohol and 1 g metal sodium were introduced under CO atmosphere into a 100 ml reactor equipped with a magnetic stirrer, a thermometer, a condenser and a dropping funnel. When all the sodium had reacted, 7.14 g 1-chloronaphthalene were introduced. The temperature was set at 25°C and a solution of 0.103 g (ethoxy carbonyl)-methyl-cobalt tetracarbonyl

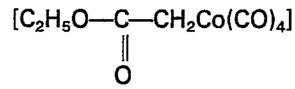

in 5 ml ethyl ether were introduced through the dropping funnel for one hour.

The solution was left under stirring at 25°C until carbon monoxide absorption ceased (approx. 2 hours), then the reaction mixture was saponified by adding 3 g NaOH and 20 ml water and maintaining boiling for approximately 2 hours. After cooling, water was added, acidification was carried out with concentrated HCl and extraction was carried out with ether. The organic phase was extracted with bicarbonate-saturated solution and then the aqueous layer was re-acidified and finally extracted with ether. By evaporation of the solvent under vacuum, 3.75 g 1-naphthoic acid were obtained. The remainder was non-reacted product.

The number of CO moles introduced into the product per Co mole was 54.7.

Examples 2—25

Using the same equipment was described in Example 1, Examples 2—25 were carried out wherein the relevant reactants and reaction conditions are given in the following Table.

| Example | Halide (g) | Catalyst (g) | Solvent | Acidity-acceptor compound | Temperature (°C) | Product (g) | Number of CO moles introduced into the product per Co mole |
|---|---|---|---|---|---|---|---|
| 2 | 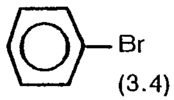 (3.4) | $C_2H_5OCOCH_2Co(CO)_4$ (0.08) | $CH_3OH$ | $NaOCH_3$ (2.3) | 17 | 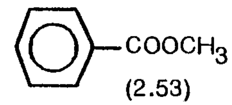 (2.53) | 26 |
| 3 | 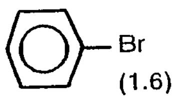 (1.6) | $FCH_2Co(CO)_4$ (0.057) | $CH_3OH$ | $NaOCH_3$ (0.82) | 22 | 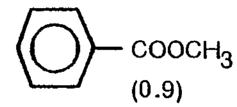 (0.9) | 24 |
| 4 | 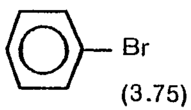 (3.75) | $C_2H_5OCOCH_2Co(CO)_4$ (0.119) | $CH_3OH$ | $NaOCH_3$ (2.5) | 25 | 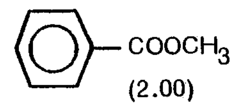 (2.00) | 32 |
| 5 | 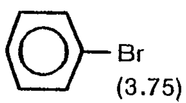 (3.75) | $C_2H_5OCOCH_2Co(CO)_4$ (0.095) | $CH_3OH$ | NaOH (2.5) | 25 | 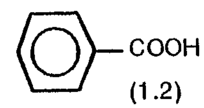 (1.2) | 27 |
| 6 | 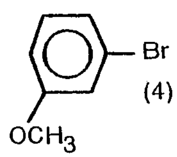 (4) | $C_2H_5OCOCH_2Co(CO)_4$ (0.072) | $CH_3OH$ | $NaOCH_3$ (2.0) | 25 | 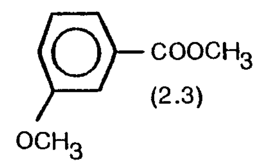 (2.3) | 49 |

| Example | Halide (g) | Catalyst (g) | Solvent | Acidity-acceptor compound | Temperature (°C) | Product (g) | Number of CO moles introduced into the product per Co mole |
|---|---|---|---|---|---|---|---|
| 7 | 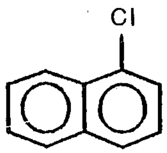 (7.14) | $FCH_2Co(CO)_4$ (0.098) | $CH_3OH$ | $NaOCH_3$ (2.3) | 25 | 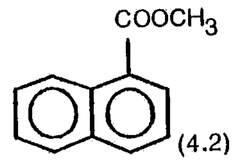 (4.2) | 47 |
| 8 | 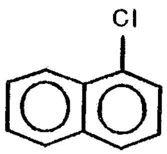 (7.14) | $FCH_2Co(CO)_4$ (0.112) | $C_2H_5OH$ | $NaOC_2H_5$ (3.0) | 25 | 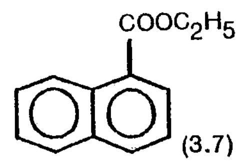 (3.7) | 34 |
| 9 | 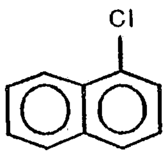 (7.14) | $C_2H_5OCOCH_2Co(CO)_4$ (0.119) | $C_2H_5OH$ | $NaOC_2H_5$ (3.0) | 25 | 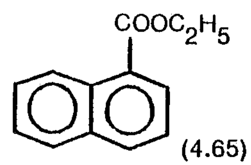 (4.65) | 50.4 |
| 10 | 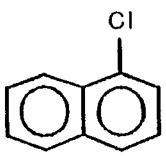 (7.14) | $C_2H_5OCOCH_2Co(CO)_4$ (0.115) | $i.C_3H_7OH$ | $NaOC_3H_7$ (3.6) | 35 | 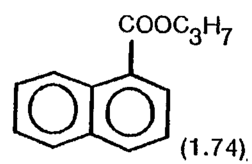 (1.74) | 18.5 |
| 11 | 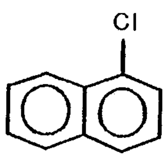 (7.14) | 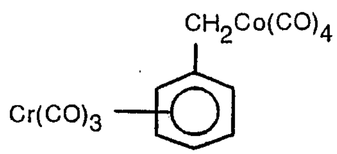 (0.066) | $CH_3OH$ | $NaOCH_3$ (2.3) | 25 | 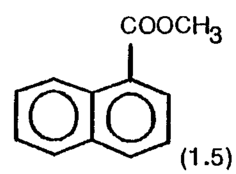 (1.5) | 49 |

| Example | Halide (g) | Catalyst (g) | Solvent | Acidity-acceptor compound | Temperature (°C) | Product (g) | Number of CO moles introduced into the product per Co mole |
|---|---|---|---|---|---|---|---|
| 12 | 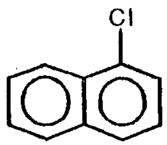 (7.14) | 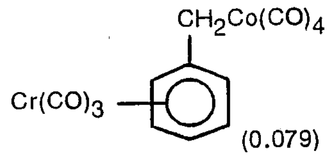 (0.079) | $CH_3OH$ | $NaOCH_3$ (2.3) | 10 | 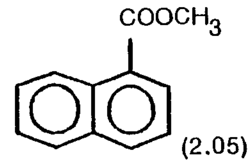 (2.05) | 56 |
| 13 | 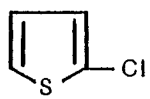 (3.2) | $C_2H_5OCOCH_2Co(CO)_4$ (0.083) | $CH_3OH$ | $NaOCH_3$ (2.3) | 25 | 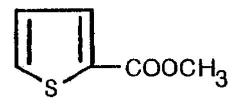 (3.0) | 65 |
| 14 | 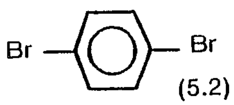 (5.2) | $C_2H_5OCOCH_2Co(CO)_4$ (0.114) | $CH_3OH$ | $NaOCH_3$ (2.6) | 25 | * 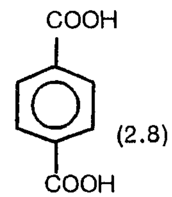 (2.8) | 60 |
| 15 | 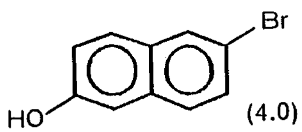 (4.0) | $C_2H_5OCOCH_2Co(CO)_4$ (0.055) | $CH_3OH$ | $NaOCH_3$ (2.3) | 45 | 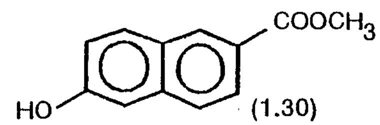 (1.30) | 30 |
| 16 | 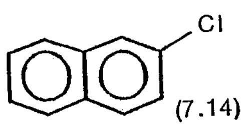 (7.14) | $C_2H_5OCOCH_2Co(CO)_4$ (0.178) | $CH_3OH$ | $K_2CO_3$ (7.5) | 25 | 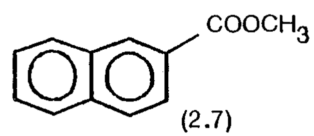 (2.7) | 21 |

| Example | Halide (g) | Catalyst (g) | Solvent | Acidity-acceptor compound | Temperature (°C) | Product (g) | Number of CO moles introduced into the product per Co mole |
|---|---|---|---|---|---|---|---|
| 17 | 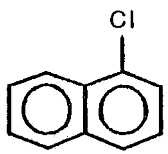 (7.14) | $C_2H_5OCOCH_2Co(CO)_4$ (0.060) | $CH_3OH$ | $NaOCH_3$ (2.3) | 0 | 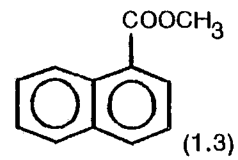 (1.3) | 30 |
| 18 | 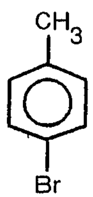 (5.95) | $C_2H_5OCOCH_2Co(CO)_4$ (0.094) | $CH_3OH$ | $NaOCH_3$ (2.1) | 25 | 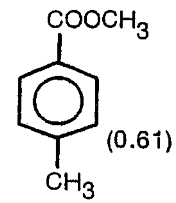 (0.61) | 11.1 |
| 19 | 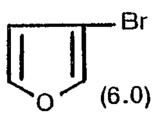 (6.0) | $C_2H_5OCOCH_2Co(CO)_4$ (0.073) | $CH_3OH$ | $NaOCH_3$ (2.3) | 25 | 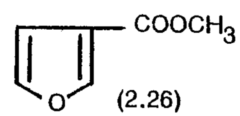 (2.26) | 56.4 |
| 20 | 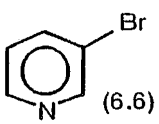 (6.6) | $C_2H_5OCOCH_2Co(CO)_4$ (0.087) | $CH_3OH$ | $NaOCH_3$ (2.3) | 25 | 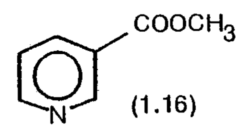 (1.16) | 24.8 |

| Example | Halide (g) | Catalyst (g) | Solvent | Acidity-acceptor compound | Temperature (°C) | Product (g) | Number of CO moles introduced into the product per Co mole |
|---|---|---|---|---|---|---|---|
| 21 | 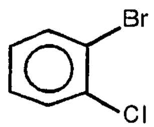 (8.6) | $C_2H_5OCOCH_2Co(CO)_4$ (0.083) | $CH_3OH$ | $NaOCH_3$ (2.3) | 25 | 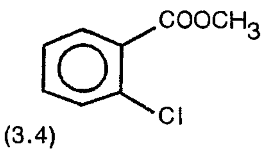 (3.4) 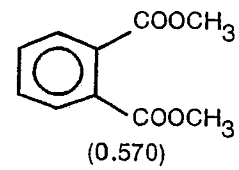 (0.570) | 80 |
| 22 | 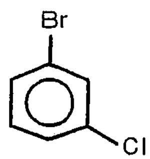 (8.6) | $C_2H_5OCOCH_2Co(CO)_4$ (0.042) | $CH_3OH$ | $NaOCH_3$ (3.05) | 25 | 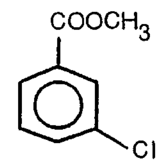 (6.0) | 216 |
| 23 | 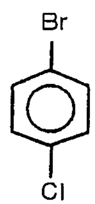 (8.6) | $C_2H_5OCOCH_2Co(CO)_4$ (0.074) | $CH_3OH$ | $NaOCH_3$ (3.05) | 25 | 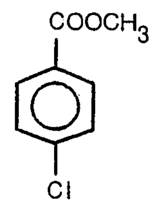 (5.88) 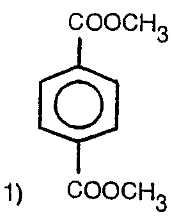 (0.11) | 125 |

| Example | Halide (g) | Catalyst (g) | Solvent | Acidity-acceptor compound | Temperature (°C) | Product (g) | Number of CO moles introduced into the product per Co mole |
|---|---|---|---|---|---|---|---|
| 24 | 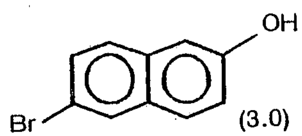 (3.0) | $C_2H_5OCOCH_2Co(CO)_4$ (0.115) | $CH_3OH$ | NaOH (2.0) | 25 | 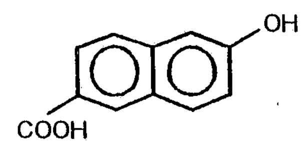 (1.2) | 14 |
| 25 | 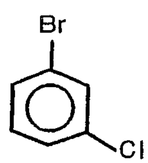 (8.6) | $CNCH_2Co(CO)_4$ (0.049) | $CH_3OH$ | $NaOCH_3$ (3.05) | 25 | 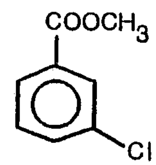 (3.26) | 82 |

* after saponification (see Example 14)

**Claims**

1. A process for the preparation of esters or salts of aromatic or etheroaromatic acids of formula (I):

$$Y—Ar—CO—OR \quad (I)$$

where:

Ar represents an aromatic group constituted by one or more benzene rings, optionally condensed, or by an etheroaromatic nucleus optionally condensed with one or more benzene rings;

Y represents zero, one or more substituents, which may be the same or different, selected from a halogen; an alkyl group having up to 6 carbon atoms; an alkoxy group having up to 5 carbon atoms; an ester group —COOR′ where R′ contains up to 5 carbon atoms; a hydroxyl group; a phenyloxy group optionally substituted with groups inert under reaction conditions; a trifluoromethyl group; a nitrile group; an amidic group ($—CONH_2$); an acetamidic group ($—NH—CO—CH_3$) or an acyl group —CO—R″ where R″ represents a hydrocarbon group having up to 8 carbon atoms; and

R represents an alkyl group $R_1$ having up to 5 carbon atoms or an alkali or alkaline-earth metal M; characterized in that a halide of formula (II)

$$Y—Ar—X \quad (II)$$

where Ar and Y have the hereinabove defined meaning and where X is Cl, Br or I, is reacted with carbon monoxide in an alcoholic solvent $R_1OH$ (where $R_1$ is an alkyl group having up to 5 carbon atoms) at atmospheric pressure and at a temperature from −10°C to 60°C in the presence of an acidity-acceptor compound and of a catalyst constituted by a cobalt complex of formula (III):

$$Z—Co(CO)_4 \quad (III)$$

where Z is a group selected from $CH_3$; $CH_2F$; $CHF_3$; $CH_2—CN$; $CH_2—COOR'''$ where R‴ is an alkyl group having up to 8 carbon atoms or a benzene group, the latter being optionally substituted with groups inert under reaction conditions; and $CH_2—Ar'$ where Ar′ is an aromatic group constituted by from one to three benzene rings optionally condensed and optionally substituted with groups inert under reaction conditions, in particular with electron-attractor groups.

2. A process as claimed in claim 1, characterised in that the acidity-acceptor compound is an alkali or alkaline-earth metal oxide or hydroxide and in that a salt is obtained.

3. A process as claimed in claim 1, characterised in that the acidity-acceptor compound is $Na_2CO_3$ or $K_2CO_3$ and in that an ester is obtained.

4. A process as claimed in claim 1, characterised in that the acidity-acceptor compound is an alcoholate $R_1O^-$ derived from alcohol $R_1OH$ used as solvent and in that an ester is obtained.

5. A process as claimed in any of claims 1 to 4, characterised in that the alcohol $R_1OH$ is selected from methyl, ethyl and isopropyl alcohol.

6. A process as claimed in any of claims 1 to 5, characterised in that the reaction is performed at a temperature from 0° to 40°C.

7. A process as claimed in any of claims 1 to 6, characterised in that the catalyst, dissolved in a solvent, is added gradually into the reactor containing the other reagents and kept under CO atmosphere.

8. A process as claimed in claim 7, characterised in that the catalyst solvent is an ether, a hydrocarbon or an alcohol $R_1OH$.

9. A process as claimed in claim 8, characterised in that the ether is ethyl ether.

10. A process as claimed in any of claims 1 to 9, characterised in that the molar ratio between halide (II) and catalyst (III) is from 20:1 to 300:1.

11. A process as claimed in claim 10, characterised in that the molar ratio between halide (II) and catalyst (III) is from 20:1 to 200:1.

12. A process as claimed in any of claims 1 to 11, characterised in that the molar ratio between the acidity-acceptor compound and halide (II) is from the stoichiometric value to 50% excess with respect to the stoichiometric value.

13. Esters or salts of aromatic or etheroaromatic acids of formula Y—Ar—CO—OR (I), wherein Ar, Y and R are as defined in claim 1, characterised by being prepared by a process as claimed in any of claims 1 to 12.

14. A process for the preparation of aromatic or etheroaromatic acids of formula

$$Y—Ar—COOH$$

where Ar and Y are as defined in claim 1, characterised in that an ester or salt of an aromatic or etheroaromatic acid prepared by the process as claimed in any of claims 1 to 12 is further treated to obtain the corresponding acid.

**Patentansprüche**

1. Verfahren zur Herstellung von Estern oder Salzen aromatischer oder heteroaromatischer Säuren der Formel (I)

$$Y—Ar—CO—OR \quad (I)$$

worin

Ar eine aromatische Gruppe, die durch einen oder mehrere, gegebenenfalls kondensierte, Benzolringe oder durch einen, gegebenenfalls mit einem oder mehreren Benzolringen kondensierten, heteroaromatischen Kern gebildet ist,

Y = 0,1 oder mehrere Substituenten bedeutet, die gleich oder verschieden sein können und ausgewählt sind aus: Halogen, einer Alkylgruppe mit bis zu 6 Kohlenstoffatomen, einer Alkoxygruppe mit bis zu 5 Kohlenstoffatomen, einer Estergruppe —COOR′, worin R′ bis zu 5 Kohlenstoffatome enthält, einer Hydroxylgruppe, einer Phenyloxygruppe, die gegebenenfalls mit unter Reaktionsbedingungen inerten Gruppen substituiert sein kann, einer Trifluormethylgruppe, einer Nitrilgruppe, einer Amidgruppe (—$CONH_2$), einer Acetamidgruppe (—NH—CO—$CH_3$) oder einer Acylgruppe —CO—R″, worin R″ eine Kohlenwasserstoffgruppe mit bis zu 8 Kohlenstoffatomen ist; und

R eine Alkylgruppe $R_1$ mit bis zu 5 Kohlenstoffatomen oder ein Alkali- oder Erdalkalimetall M ist; dadurch gekennzeichnet, daß ein Halogenid der Formel (II)

Y—Ar—X (II)

worin Ar und Y die oben definierte Bedeutung haben und X = Cl, Br oder J ist, mit Kohlenmonoxid in einem alkoholischen Lösungsmittel $R_1OH$ (worin $R_1$ eine Alkylgruppe mit bis zu 5 Kohlenstoffatomen ist) bei atmosphärischem Druck und bei einer Temperatur von −10 bis 60°C in Anwesenheit einer Säureakzeptorverbindung und eines Katalysators, der aus einem Kobaltkomplex der Formel (III)

Z—$Co(CO)_4$ (III)

besteht, worin Z eine Gruppe, ausgewählt von $CH_3$, $CH_2F$, $CHF_2$, $CF_3$, $CH_2$—CN, $CH_2$—COOR‴, worin R‴ eine Alkylgruppe mit bis zu 8 Kohlenstoffatomen oder eine Benzolgruppe ist, wobei letztere gegebenenfalls mit unter Reaktionsbedingungen inerten Gruppen substituiert sein kann, bzw. $CH_2$—Ar′, worin Ar′ eine aromatische Gruppe, die aus einem bis drei, gegebenenfalls kondensierten Benzolringen besteht und gegebenenfalls mit unter Reaktionsbedingungen inerten Gruppen, insbesondere mit Elektronen anziehenden Gruppen, substituiert sein kann, umgesetzt wird.

2. Verfahren nach Anspruch 1, dadurch gekennzeichnet, daß die Säureakzeptorverbindung ein Alkali- oder Erdalkalimetalloxid oder -hydroxid ist und daß ein Salz erhalten wird.

3. Verfahren nach Anspruch 1, dadurch gekennzeichnet, daß die Säureakzeptorverbindung $Na_2CO_3$ oder $K_2CO_3$ ist und ein Ester erhalten wird.

4. Verfahren nach Anspruch 1, dadurch gekennzeichnet, daß die Säureakzeptorverbindung ein Alkoholat $R_1O^-$, abgeleitet von dem als Lösungsmittel verwendeten Alkohol $R_1OH$, ist und ein Ester erhalten wird.

5. Verfahren nach einem der Ansprüche 1 bis 4, dadurch gekennzeichnet, daß der Alkohol $R_1OH$ ausgewählt ist von Methyl-, Ethyl- und Isopropylalkohol.

6. Verfahren nach einem der Ansprüche 1 bis 5, dadurch gekennzeichnet, daß die Reaktion bei einer Temperatur von 0 bis 40°C durchgeführt wird.

7. Verfahren nach einem der Ansprüche 1 bis 6, dadurch gekennzeichnet, daß der in einem Lösungsmittel gelöste Katalysator allmählich in das Reaktionsgefäß eingeführt wird, der die anderen Reaktanten enthält und unter einer CO-Atmosphäre gehalten wird.

8. Verfahren nach Anspruch 7, dadurch gekennzeichnet, daß das Katalysatorlösungsmittel ein Ether, ein Kohlenwasserstoff oder ein Alkohol $R_1OH$ ist.

9. Verfahren nach Anspruch 8, dadurch gekennzeichnet, daß der Ether Ethylether ist.

10. Verfahren nach einem der Ansprüche 1 bis 9, dadurch gekennzeichnet, daß das molare Verhältnis zwischen Halogenid (II) und Katalysator (III) von 20:1 bis 300:1 beträgt.

11. Verfahren nach Anspruch 10, dadurch gekennzeichnet, daß das molare Verhältnis zwischen Halogenid (II) und Katalysator (III) von 20:1 bis 200:1 beträgt.

12. Verfahren nach einem der Ansprüche 1 bis 11, dadurch gekennzeichnet, daß das molare Verhältnis zwischen der Säureakzeptorverbindung und dem Halogenid (II) zwischen dem stöchiometrischen Wert bis zu einem 50-%igen Überschuß, bezogen auf den stöchiometrischen Wert, liegt.

13. Ester oder Salze aromatischer oder heteroaromatischer Säuren der Formel Y—Ar—CO—OR (I), worin Ar, Y und R wie in Anspruch 1 definiert sind, dadurch gekennzeichnet, daß sie nach einem Verfahren gemäß einem der Ansprüche 1 bis 12 hergestellt sind.

14. Verfahren zur Herstellung aromatischer oder heteroaromatischer Säuren der Formel:

Y—Ar—COOH

worin Ar und Y wie in Anspruch 1 definiert sind, dadurch gekennzeichnet, daß ein Ester oder Salz einer aromatischen oder heteroaromatischen Säure, hergestellt nach dem Verfahren gemäß einem der Ansprüche 1 bis 12, weiter unter Bildung der entsprechenden Säure behandelt wird.

**Revendications**

1. Procédé de préparation d'esters ou de sels d'acides aromatiques ou hétéro-aromatiques de formule (I):

Y—AR—CO—OR (I)

dans laquelle:

Ar représente un groupe aromatique constitué par un ou plusieurs cycles aromatiques, éventuellement condensés, ou par un noyau hétéro-aromatique éventuellement condensé avec un ou plusieurs cycles benzéniques;

Y représente zéro, un seul ou plusieurs substituants qui peuvent être identiques ou différents, choisis parmi un halogène; un groupe alkyle possédant jusqu'à 6 atomes de carbone; un groupe alkoxy possédant jusqu'à 5 atomes de carbone; un groupe ester —COOR′ dans lequel R′ contient jusqu'à 5 atomes de carbone; un groupe hydroxyle; un groupe phényloxy éventuellement substitué par des groupes inertes dans les conditions de la réaction; un groupe trifluorométhyle; un groupe nitrile; un groupe amide (—$CONH_2$);

un groupe acétamido (—NH—CO—$CH_3$) ou un groupe acyle —CO—R″ dans lequel R″ représente un groupe hydrocarboné possédant jusqu'à 8 atomes de carbone; et

R représente un groupe alkyle $R_1$ possédant jusqu'à 5 atomes de carbone ou un métal M alcalin ou alcalino-terreux; caractérisé en ce que l'on fait réagir un halogénure de formule (II):

$$Y—Ar—X \qquad (II)$$

dans laquelle:

Ar et Y ont les significations indiquées ci-dessus, et dans laquelle:

X représente Cl, Br ou I, avec un monoxyde de carbone dans un solvant alcoolique $R_1OH$ (dans lequel $R_1$ représente un groupe alkyle possédant jusqu'à 5 atomes de carbone) à la pression atmosphérique et à une température comprise entre −10°C à 60°C en présence d'un composé accepteur d'acide et d'un catalyseur constitué d'un complexe de cobalt de formule (III):

$$Z—Co(CO)_4 \qquad (III)$$

dans laquelle:

Z représente un groupe choisi parmi $CH_3$; $CH_2F$; $CHF_2$; $CF_3$; $CH_2CN$; $CH_2$—COOR‴ où R‴ représente un groupe alkyle possédant jusqu'à 8 atomes de carbone ou un groupe benzénique, ce dernier étant éventuellement substitué par des groupes inertes dans les conditions de la réaction; et $CH_2Ar'$ dans lequel Ar' représente un groupe aromatique constitué par 1 à 3 cycles benzéniques éventuellement condensés et éventuellement substitués par des groupes inertes dans les conditions de la réaction, en particulier par des groupes attracteurs d'électrons.

2. Procédé selon la revendication 1, caractérisé en ce que le composé accepteur d'acidité est un oxyde ou hydroxyde de métal alcalin ou alcalino-terreux et en ce que l'on obtient un sel.

3. Procédé selon la revendication 1, caractérisé en ce que le composé accepteur d'acidité est $Na_2CO_3$ ou $K_2CO_3$ et en ce que l'on obtient un ester.

4. Composé selon la revendication 1, caractérisé en ce que le composé accepteur d'acidité est un alcoolate, $R_1O^-$ dérivé d'un alcool $R_1OH$ utilisé comme solvant et en ce que l'on obtient un ester.

5. Procédé selon l'une quelconque des revendications 1 à 4, caractérisé en ce que l'alcool $R_1OH$ est choisi parmi les alcools méthylique, éthylique et isopropylique.

6. Procédé selon l'une quelconque des revendications 1 à 5, caractérisé en ce que l'on met la réaction en oeuvre à une température de 0°C à 40°C.

7. Procédé selon l'une quelconque des revendications 1 à 6, caractérisé en ce que le catalyseur, dissous dans un solvant, est ajouté progressivement dans le réacteur qui contient les autres réactifs et qui est maintenu sous atmosphère de CO.

8. Procédé selon la revendication 7, caractérisé en ce que le solvant du catalyseur est un éther, un hydrocarbure ou un alcool $R_1OH$.

9. Procédé selon la revendication 8, caractérisé en ce que l'éther est l'éther éthylique.

10. Procédé selon l'une quelconque des revendications 1 à 9, caractérisé en ce que le rapport molaire entre l'halogénure (II) et le catalyseur (III) est compris entre 20/1 et 300/1.

11. Procédé selon la revendication 10, caractérisé en ce que le rapport molaire entre l'halogénure (II) et le catalyseur (III) est compris entre 20/1 et 200/1.

12. Procédé selon l'une quelconque des revendications 1 à 11, caractérisé en ce que le rapport molaire entre le composé accepteur d'acidité et l'halogénure (II) est compris entre celui correspondant à la stoéchiométrie et celui correspondant à un excès de 50% par rapport à la stoéchiométrie.

13. Esters ou sels d'acides aromatiques ou hétéro-aromatiques de formule Y—Ar—CO—OR (I) dans laquelle Ar, Y et R sont tels que définis dans la revendication 1, caractérisés en ce qu'on les prépare par un procéde selon l'une quelconque des revendications 1 à 12.

14. Procédé de préparation d'acides aromatiques ou hétéro-aromatiques de formule Y—Ar—COOH dans laquelle Ar et Y sont tels que définis dans la revendication 1, caractérisé en ce que un ester ou un sel d'un acide aromatique ou hétéro-aromatique préparé par le procédé selon l'une des revendications 1 à 12, est traité ultérieurement pour obtenir l'acide correspondant.